# EUROPEAN PATENT APPLICATION

(11) **EP 4 501 374 A1**
(43) Date of publication of application: **05.02.2025**
(21) Application number: 23823504.8
(22) Date of filing: 12.04.2023
(51) Int. Cl.: A61M 5/142, A61M 5/20

(54) **PUNCTURE DEVICE AND MEDICINAL SOLUTION ADMINISTERING DEVICE EQUIPPED WITH PUNCTURE DEVICE**

(30) Priority: 17.06.2022 JP 2022098085
(71) Applicant: Terumo Kabushiki Kaisha, Tokyo 151-0072 (JP)
(72) Inventor: YANAGISAWA, Katsuya, Ashigarakami-gun, Kanagawa 259-0151 (JP); HASEGAWA, Makoto, Ashigarakami-gun, Kanagawa 259-0151 (JP)
(74) Representative: Casalonga
(86) International application number: PCT/JP2023/014831
(87) International publication number: WO 2023/243201

(57) **Abstract**

In a housing (14) of a puncture device (10), an abutting member (68) that has an abutting surface (106) having contact with skin (S) of a patient is movably disposed. The abutting member (68) is provided so as to protrude from a contact surface (26) of the housing (14). A needle member (66) relatively moves with respect to the abutting member (68) so as to protrude from the abutting surface (106) toward the skin (S). When a relative position between the housing (14) and a facing portion (S1) of the abutting surface (106) of the skin (S) changes in a state where the contact surface (26) of the housing (14) has contact with the skin (S), the abutting member (68) relatively moves with respect to the housing (14) so as to maintain the contact between the abutting surface (106) and the skin (S).

## Description

### Technical Field

The present invention relates to a puncture device including a needle member that punctures skin of a living body and a medicine solution administration device including the puncture device.

### Background Art

JP 2004-208726 A discloses a puncture device that punctures skin of a user (patient) to be administered with a puncture needle. The puncture device includes a main body portion, a puncture portion that is housed in the main body portion, and a moving body that holds the puncture portion. The puncture portion includes a needle at a distal end and is held on a bottom surface of the moving body. The puncture portion is movably disposed toward a bottom wall side of the main body portion together with the moving body.

After the bottom wall of the main body portion is brought into close contact with the skin of the user, by moving the puncture portion toward the bottom wall together with the moving body, the needle protrudes from the bottom wall and punctures the skin.

### Summary of Invention

In a state where skin is punctured by a needle, a bottom wall of a main body portion and the skin are separated when a user moves, and accordingly, a space is generated between a puncture device and the skin, and the needle is easily removed from the skin.

An object of the present invention is to solve the above problems.

An aspect of the present invention is a puncture device including
a housing having a contact surface that has contact with skin of a living body,
an abutting member that has an abutting surface that has contact with the skin, is movably provided in the housing, the abutting surface being protrudable from the contact surface, and
a needle member that includes a puncture needle and is relatively movable with respect to the abutting member, the puncture needle being protrudable from the abutting surface toward the skin, wherein
when a relative position between the housing and a portion, facing the abutting surface, of the skin changes in a state where the housing has contact with the skin, the abutting member relatively moves with respect to the housing so as to maintain the contact between the abutting surface and the skin.

According to the present invention, even in a case where a relative position between a housing and skin changes due to a body movement of a living body, in a state where the skin of the living body is punctured with a puncture needle, since an abutting member relatively moves with respect to the housing following the skin, removal of the puncture needle from the skin is suitably prevented.

### Brief Description of Drawings

Fig. 1 is a partially-omitted perspective view of a medicine solution administration device including a puncture device according to an embodiment of the present invention.
Fig. 2 is an enlarged plan view of vicinity of the puncture device of the medicine solution administration device in Fig. 1.
Fig. 3 is a cross-sectional view of the puncture device illustrated in Fig. 2.
Fig. 4 is an exploded perspective view of the puncture device illustrated in Fig. 3.
Fig. 5 is a cross-sectional view of a housing distal portion taken along a V-V line in Fig. 2.
Fig. 6 is a cross-sectional view taken along a VI-VI line in Fig. 3.
Fig. 7 is a partial cross-sectional perspective view of the puncture device illustrated in Fig. 4.
Fig. 8 is an overall front view of the puncture device illustrated in Fig. 7.
Fig. 9 is a cross-sectional view of a state in which a housing of the medicine solution administration device illustrated in Fig. 3 is brought into contact with skin of a patient.
Fig. 10 is a partial cross-sectional perspective view of a puncture mechanism at the time of puncture.
Fig. 11 is a cross-sectional plan view of an abutting member in Fig. 10 viewed from above.
Fig. 12 is a cross-sectional view illustrating the time of puncture with a puncture needle.
Fig. 13 is a cross-sectional view illustrating a state where the housing is separated from the skin at the time of puncture by the puncture device illustrated in Fig. 12.

### Description of Embodiments

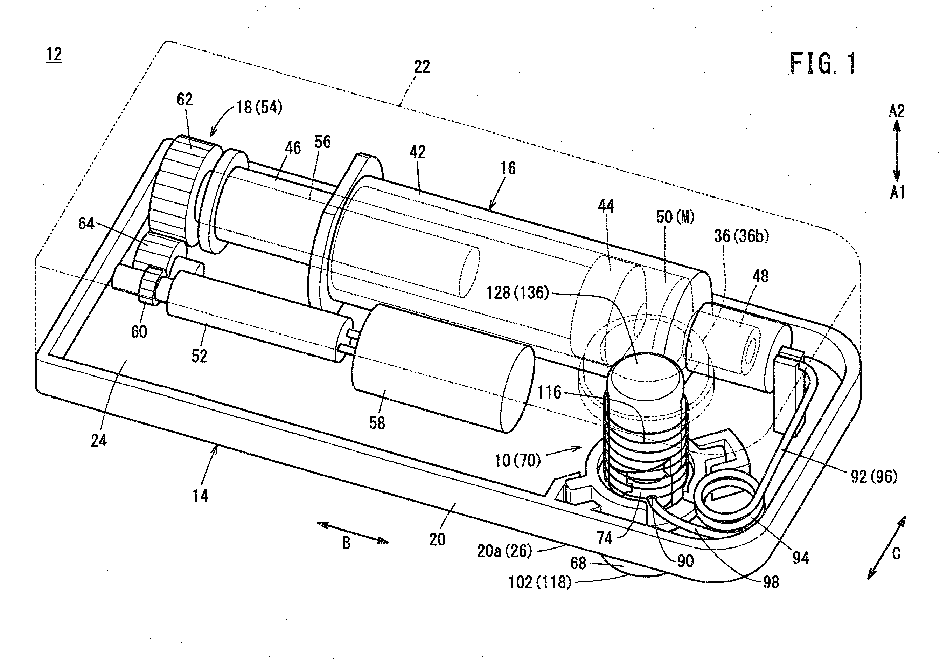

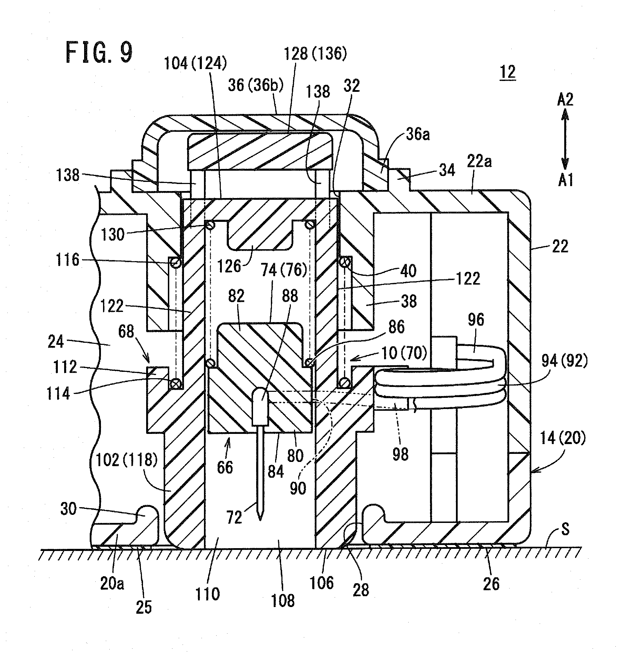

As illustrated in Fig. 1, a puncture device 10 according to the present embodiment is used for a medicine solution administration device 12 that can administer a medicine solution M. The medicine solution administration device 12 is used, for example, to be brought into contact with skin S (refer to Fig. 9) of a patient and administer the medicine solution M subcutaneously to the patient.

As illustrated in Fig. 1, the medicine solution administration device 12 includes a housing 14, a prefilled syringe 16 that stores the medicine solution M, a movement mechanism 18 that delivers the medicine solution M in the prefilled syringe 16, and the puncture device 10 that can puncture the skin S (refer to Fig. 9) of the patient.

The housing 14 serves as both of an enclosure of the medicine solution administration device 12 and an enclosure of the puncture device 10. That is, the medicine solution administration device 12 and the puncture device 10 share the housing 14.

The housing 14 includes a housing main body 20 and a housing cover 22. When the medicine solution administration device 12 including the puncture device 10 administers the medicine solution M, the housing main body 20 is disposed on the skin S of the patient, and the housing cover 22 is disposed on an opposite side to the skin S with the housing main body 20 therebetween (refer to Fig. 9). The housing main body 20 and the housing cover 22 overlap in a thickness direction (arrow A1 and A2 directions) of the housing 14. The arrow A1 direction is a direction toward the skin S when the medicine solution administration device 12 is installed in the skin S of the patient. The arrow A2 direction is a direction away from the skin S when the medicine solution administration device 12 is installed in the skin S of the patient. Hereinafter, for convenience of description, a direction from the housing cover 22 toward the housing main body 20 (arrow A1 direction) is referred to as downward, and a direction from the housing main body 20 toward the housing cover 22 (arrow A2 direction) is referred to as upward.

In a top view of the medicine solution administration device 12, the housing 14 has a rectangular shape elongated in a longitudinal direction (arrow B direction). The housing cover 22 covers an upper portion of the housing main body 20. The housing main body 20 and the housing cover 22 can be separated in a vertical direction (direction of arrows A1 and A2 in Fig. 1).

The housing 14 has a space 24 surrounded by the housing main body 20 and the housing cover 22, and the prefilled syringe 16, the movement mechanism 18, and the puncture device 10 are housed in the space 24 of the housing 14.

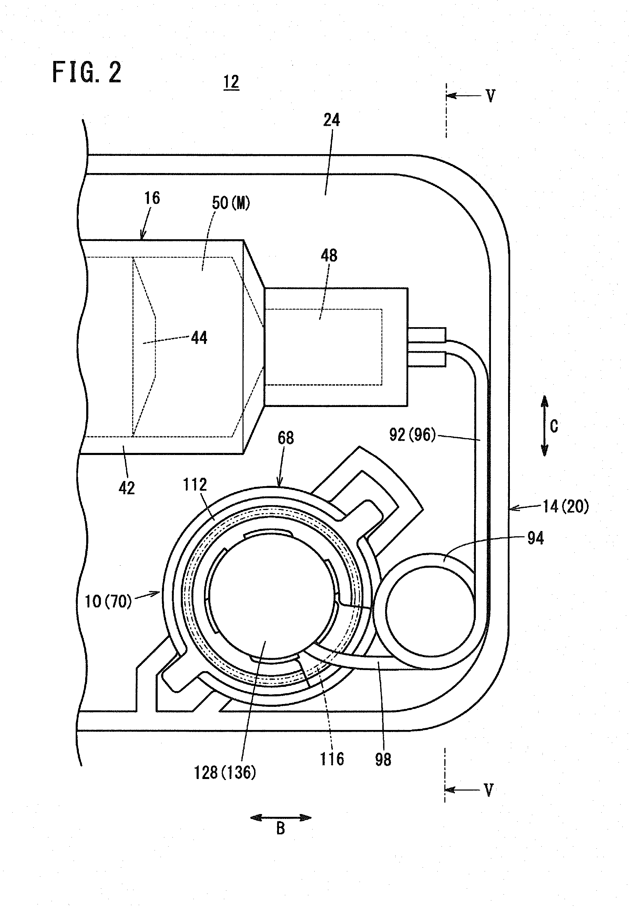

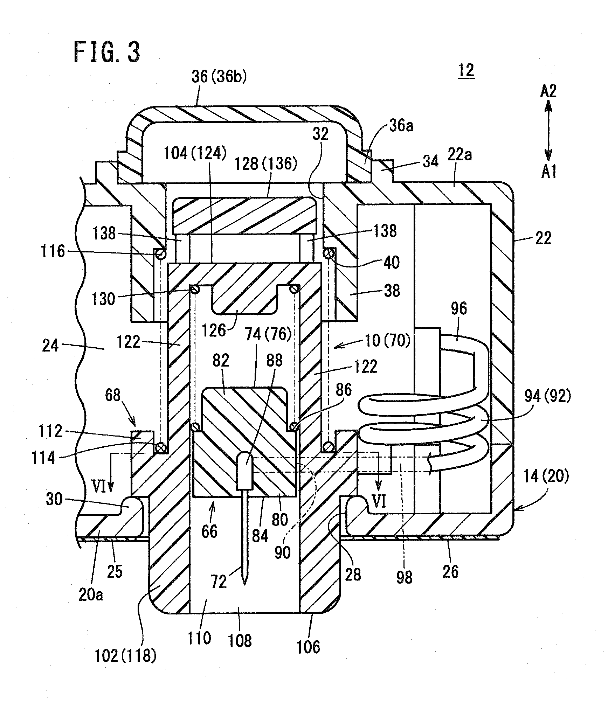

As illustrated in Fig. 3, a bottom wall 20a of the housing main body 20 has a contact surface 26 having contact with the skin S of the patient. In the present embodiment, the housing 14 has a bonding material 25 fixed to the bottom wall 20a of the housing main body 20, and one surface of the bonding material 25 is the contact surface 26. The contact surface 26 is flat along the longitudinal direction (arrow B direction in Fig. 2) of the housing 14 and a width direction (arrow C direction in Fig. 2) orthogonal to the longitudinal direction. The bottom wall 20a has a first hole portion 28. The first hole portion 28 is disposed at a distal end portion along the longitudinal direction of the housing main body 20. The first hole portion 28 passes through the bottom wall 20a and communicates outside of the housing main body 20 with the space 24.

The bottom wall 20a of the housing main body 20 has a stopper portion 30. The stopper portion 30 protrudes from the bottom wall 20a toward the housing cover 22 and is formed in an annular shape surrounding the first hole portion 28. That is, the stopper portion 30 is disposed on an outer periphery of the first hole portion 28.

An upper wall 22a of the housing cover 22 is formed in parallel with the bottom wall 20a of the housing main body 20. The upper wall 22a of the housing cover 22 has a second hole portion 32. The second hole portion 32 is disposed at a distal end portion of the housing cover 22. In a thickness direction of the housing 14, the first hole portion 28 and the second hole portion 32 face each other. The upper wall 22a has an annular protrusion portion 34 surrounding the second hole portion 32. The annular protrusion portion 34 protrudes upward (arrow A2 direction) from the upper wall 22a. The annular protrusion portion 34 is disposed to be separated radially outward from the second hole portion 32.

On the upper wall 22a of the housing cover 22, a cover member 36 that is elastically deformable is disposed. The cover member 36 has a cap-like shape. The cover member 36 is disposed in the annular protrusion portion 34. The cover member 36 has an outer edge portion 36a and a covering portion 36b disposed on an inner side of the outer edge portion 36a. The outer edge portion 36a abuts on the upper wall 22a of the housing cover 22 adjacent to an inner peripheral surface of the annular protrusion portion 34.

The covering portion 36b offsets in an axial direction (arrow A2 direction) with respect to the outer edge portion 36a and faces the second hole portion 32. The covering portion 36b is disposed to be separated upward (arrow A2 direction) from the upper wall 22a of the housing cover 22. The covering portion 36b protrudes upward (arrow A2 direction) with respect to the annular protrusion portion 34. The second hole portion 32 is covered with the cover member 36.

The upper wall 22a of the housing cover 22 has a support portion 38. The support portion 38 has a cylindrical shape that protrudes from the upper wall 22a toward the housing main body 20 and surrounds the second hole portion 32. The second hole portion 32 passes through from the upper wall 22a to a lower end of the support portion 38. An inner peripheral surface of the support portion 38 has a housing-side receiving portion 40 recessed radially outward. The housing-side receiving portion 40 is a step formed from the lower end of the support portion 38 toward the upper wall 22a of the housing cover 22.

As illustrated in Fig. 1, the prefilled syringe 16 is housed in the housing 14 and is disposed along the longitudinal direction (arrow B direction) of the housing 14. The prefilled syringe 16 includes a barrel 42, a gasket 44, and a rod 46. The barrel 42 has a cylindrical shape along an axial direction, of which a proximal end is opened. A distal end of the barrel 42 has a nozzle 48 that protrudes in a distal end direction. The gasket 44 is movably housed in the barrel 42. The gasket 44 includes an elastic material and is liquid-tightly inserted into the barrel 42. A medicine chamber 50 filled with the medicine solution M is provided between the distal end of the barrel 42 and the gasket 44.

The rod 46 is formed in a cylindrical shape. A distal end portion of the rod 46 is inserted into the barrel 42 and is coupled to a proximal end of the gasket 44. A proximal end portion of the rod 46 is disposed outside the barrel 42. The rod 46 is disposed to be relatively movable in the axial direction (distal end direction, proximal end direction, and arrow B direction) with respect to the barrel 42.

The movement mechanism 18 includes a motor 52, a gear portion 54, and a shaft 56. The motor 52 is disposed in parallel with the prefilled syringe 16. The motor 52 is electrically connected to a power supply unit 58. The power supply unit 58 is disposed in a distal end direction of the motor 52. The power supply unit 58 can supply power to the motor 52. The power is supplied from the power supply unit 58 to the motor 52, and the power rotationally drives a driving shaft (not illustrated) of the motor 52. The motor 52 is driven and controlled by a control unit (not illustrated).

The gear portion 54 is disposed in a proximal end direction of the motor 52. The gear portion 54 includes a drive gear 60 coupled to the driving shaft, a driven gear 62 connected to the shaft 56, and an intermediate gear 64 meshed with the drive gear 60 and the driven gear 62. The intermediate gear 64 is disposed between the drive gear 60 and the driven gear 62. The drive gear 60, the driven gear 62, and the intermediate gear 64 are arranged in parallel in the width direction (arrow C direction) of the housing 14.

By rotationally driving the motor 52, the drive gear 60 rotates together with the driving shaft of the motor 52. As the drive gear 60 rotates, the intermediate gear 64 and the driven gear 62 rotate so that the shaft 56 rotates.

The shaft 56 extends along the axial direction and is inserted into the rod 46. In the rod 46, an outer peripheral surface of the shaft 56 is screwed with an inner peripheral surface of the rod 46. The shaft 56 is housed in the barrel 42 of the prefilled syringe 16 together with the rod 46. The driven gear 62 is coupled to a proximal end of the shaft 56.

When a driving force of the motor 52 is transmitted to the shaft 56 via the gear portion 54 so as to rotate the shaft 56, the rod 46 screwed with the shaft 56 moves along the axial direction (arrow B direction). The movement of the rod 46 moves the gasket 44 in the barrel 42 along the axial direction together with the rod 46, and the medicine solution M in the medicine chamber 50 is delivered to a distal end direction by the gasket 44.

The puncture device 10 is disposed at a distal end portion of the housing 14. As illustrated in Fig. 3, the puncture device 10 faces the first and second hole portions 28 and 32.

The puncture device 10 includes a needle member 66, an abutting member 68, and a puncture mechanism 70 that biases the needle member 66 toward the skin S of the patient.

The needle member 66 includes a puncture needle 72 and a needle hub 74 that holds the puncture needle 72. The puncture needle 72 is a hollow needle. A distal end of the puncture needle 72 can puncture the skin S of the patient. A proximal end of the puncture needle 72 is held at an axial center of the needle hub 74.

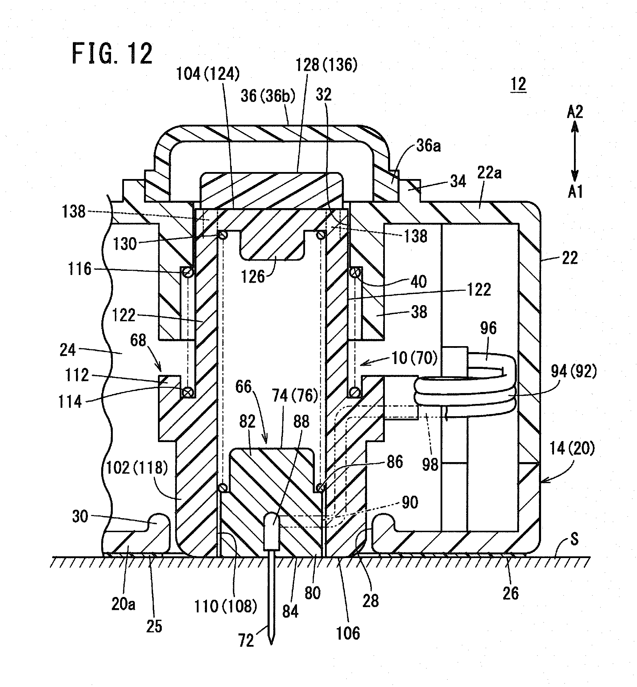

The needle hub 74 includes a hub main body 76. The hub main body 76 has a circular shape as viewed from an axial direction of the needle hub 74. The hub main body 76 includes a large diameter portion 80 and a small diameter portion 82. The large diameter portion 80 is disposed in a distal end direction (arrow A1 direction) of the hub main body 76. A distal end of the hub main body 76 (large diameter portion 80) has a hub surface 84. The hub surface 84 is a flat surface orthogonal to an axis line of the needle hub 74. The proximal end of the puncture needle 72 is held at an axial center of the hub surface 84. When the skin S of the patient is punctured by the needle member 66, the hub surface 84 is a surface having contact with the skin S (refer to Fig. 12).

The small diameter portion 82 is disposed in a proximal end direction (arrow A2 direction) of the large diameter portion 80. The diameter of the small diameter portion 82 is smaller than the diameter of the large diameter portion 80. At a boundary between the small diameter portion 82 and the large diameter portion 80, an annular hub-side receiving portion 86 extending in a radial direction is provided.

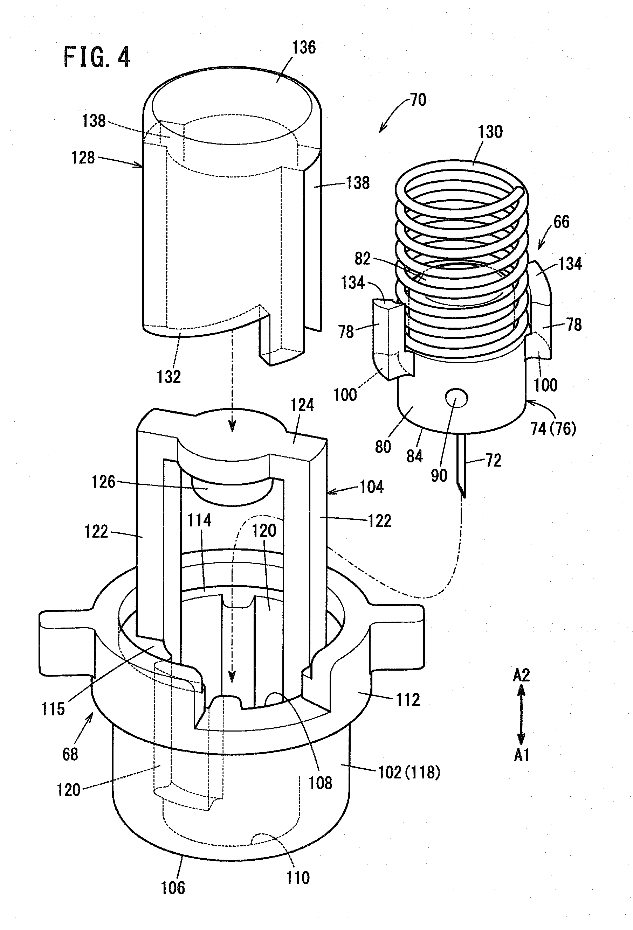

The large diameter portion 80 of the hub main body 76 includes a communication path 88. A distal end of the communication path 88 extends along an axial center of the hub main body 76 and opens to the hub surface 84. The distal end of the communication path 88 communicates with inside of the puncture needle 72. A proximal end of the communication path 88 extends outward in the radial direction and opens to an outer peripheral surface of the large diameter portion 80. The outer peripheral surface of the large diameter portion 80 has a connection hole 90 (refer to Fig. 4), and the connection hole 90 and the proximal end of the communication path 88 communicate with each other.

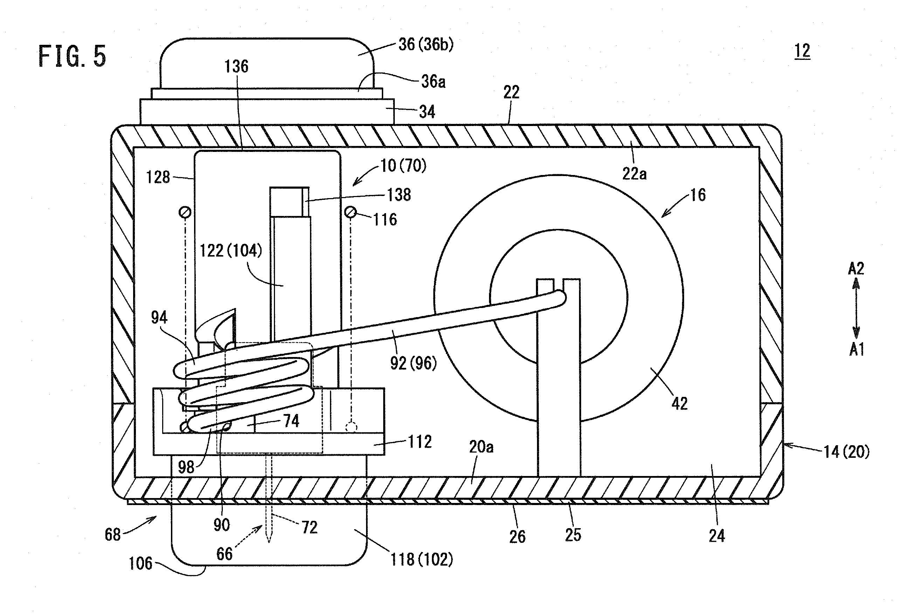

As illustrated in Fig. 5, the connection hole 90 of the needle hub 74 and the prefilled syringe 16 are connected with a tube 92 that is elastically deformable. The tube 92 is a tubular body and is disposed at the distal end portion of the housing 14 (refer to Fig. 2). The tube 92 has flexibility and can deliver the medicine solution M from the prefilled syringe 16 to the needle member 66. The medicine solution M is supplied from the prefilled syringe 16 to the communication path 88 of the needle member 66 (refer to Fig. 3) through the tube 92 and is delivered from the communication path 88 to the puncture needle 72. The tube 92 includes a spirally wound spiral portion 94, a first extending portion 96 that extends from one end of the spiral portion 94, and a second extending portion 98 that extends from another end of the spiral portion 94.

The spiral portion 94 has a substantially circular shape and wound along the vertical direction (arrow A1 and A2 directions) a plurality of times. As illustrated in Fig. 2, the first extending portion 96 substantially linearly extends along the width direction of the housing 14. An end portion of the first extending portion 96 is connected to the nozzle 48 of the prefilled syringe 16. The second extending portion 98 extends along the longitudinal direction of the housing 14. As illustrated in Fig. 5, an end portion of the second extending portion 98 is connected to the connection hole 90 of the needle hub 74. The spiral portion 94 of the tube 92 is housed in the housing 14 in a state of being bent in the vertical direction. When the needle member 66 is displaced in an axial direction (arrow A1 and A2 directions) of the needle member 66 with respect to the housing 14, the spiral portion 94 of the tube 92 is elastically deformed by moving the second extending portion 98 together with the needle member 66.

As illustrated in Fig. 4, the needle hub 74 further includes a pair of guided portions 78 disposed on a radially outer side of the hub main body 76. A distal end of the guided portion 78 is connected to the outer peripheral surface of the large diameter portion 80. The pair of guided portions 78 faces an outer peripheral surface of the small diameter portion 82 and is disposed to be radially separated outward from the outer peripheral surface of the small diameter portion 82. The one guided portion 78 and the other guided portion 78 are symmetrically arranged with respect to the axial center of the hub main body 76. Each guided portion 78 includes a locking portion 100. The locking portion 100 is disposed at the distal end of the guided portion 78. The locking portion 100 has a flat shape orthogonal to the axial direction of the needle hub 74.

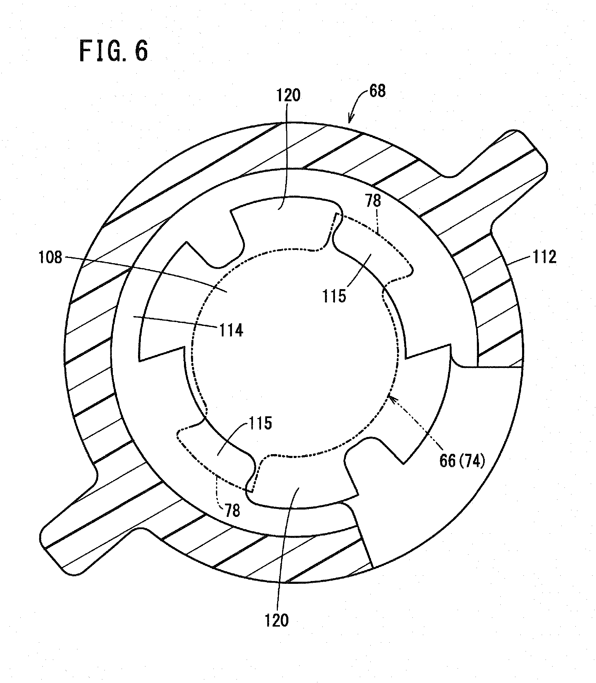

As illustrated in Fig. 6, on a cross section perpendicular to the axial direction of the needle member 66, a cross-sectional shape of the guided portion 78 is an arc-like shape.

As illustrated in Fig. 3, the abutting member 68 is movably provided in the housing 14 along the thickness direction (arrow A1 and A2 directions) of the housing 14. The abutting member 68 is disposed in the first hole portion 28 of the housing main body 20. As illustrated in Fig. 4, the abutting member 68 includes a cylindrical portion 102 and a gate portion 104 that protrudes from the cylindrical portion 102 in the proximal end direction (arrow A2 direction). The cylindrical portion 102 is disposed at a distal end portion of the abutting member 68. A distal end of the cylindrical portion 102 has an abutting surface 106 that can have contact with the skin S of the patient (refer to Fig. 9). The abutting surface 106 is a flat surface orthogonal to an axis line of the abutting member 68. As illustrated in Fig. 3, a housing space 108 in which the needle member 66 is movably housed is provided in the cylindrical portion 102. The needle member 66 can relatively move in the axial direction (arrow A1 and A2 directions) with respect to the abutting member 68, through the housing space 108.

The housing space 108 has an opening portion 110 that opens at the distal end (abutting surface 106) of the cylindrical portion 102. The puncture needle 72 can protrude from the abutting surface 106 via the opening portion 110.

A proximal end of the cylindrical portion 102 has a flange portion 112 of which a diameter is radially expanded outward. The flange portion 112 has an annular shape surrounding the housing space 108. As illustrated in Fig. 4, the flange portion 112 includes an annular spring receiving portion 114 that radially extends inward from an inner peripheral surface of the flange portion 112. The spring receiving portion 114 is disposed radially outside of the housing space 108.

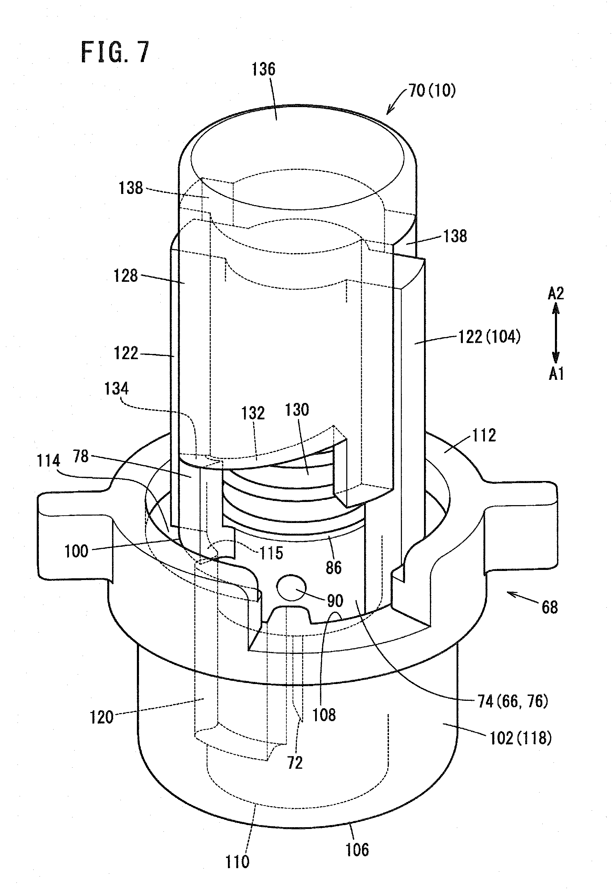

The abutting member 68 includes an engagement portion 115. The engagement portion 115 is a flat surface orthogonal to the axial direction of the abutting member 68. As illustrated in Fig. 7, in an initial state of the puncture device 10, the locking portion 100 of the needle hub 74 abuts on the engagement portion 115 of the abutting member 68. As a result, movement of the needle hub 74 in a puncture direction (distal end direction and arrow A1 direction) with respect to the abutting member 68 is prevented.

As illustrated in Fig. 3, in the housing 14, between the cylindrical portion 102 of the abutting member 68 and the support portion 38 of the housing 14, a following biasing member 116 is disposed. The following biasing member 116 is a spring member including a coil spring. The following biasing member 116 is disposed between the housing-side receiving portion 40 of the support portion 38 and the spring receiving portion 114 of the cylindrical portion 102. The small diameter portion 82 of the hub main body 76 is inserted into one end portion of the following biasing member 116. A convex portion 126 to be described later of the abutting member 68 is inserted into another end portion of the following biasing member 116. A biasing force of the following biasing member 116 is biased against the abutting member 68. The abutting member 68 is biased in the distal end direction (puncture direction and arrow A1 direction) by the biasing force of the following biasing member 116. That is, the following biasing member 116 biases the abutting member 68 toward the skin S of the patient.

When the abutting surface 106 of the abutting member 68 does not have contact with the skin S of the patient, the flange portion 112 of the abutting member 68 abuts on the stopper portion 30. The flange portion 112 of the abutting member 68 abuts on the stopper portion 30 of the housing 14 so as to prevent removal of the abutting member 68 through the first hole portion 28.

The abutting member 68 has a protrusion portion 118 that protrudes from the contact surface 26 of the housing 14 when the abutting surface 106 of the abutting member 68 does not have contact with the skin S. The protrusion portion 118 protrudes to the outside of the housing 14 through the first hole portion 28 of the housing main body 20. When the contact surface 26 of the housing 14 has contact with the skin S of the patient, the abutting surface 106 of the abutting member 68 is pressed by the skin S so that the protrusion portion 118 is housed in the housing 14 through the first hole portion 28 (refer to Fig. 9).

As illustrated in Fig. 4, the cylindrical portion 102 has a pair of guide grooves 120 that can guide the needle member 66 in the axial direction. A proximal end of the guide groove 120 is disposed at a position adjacent to the engagement portion 115 in a circumferential direction of the cylindrical portion 102. The guide groove 120 is recessed radially outward from the inner peripheral surface of the cylindrical portion 102 and faces the housing space 108. The guided portion 78 of the needle member 66 can be inserted into the guide groove 120. Each guide groove 120 extends along the axial direction (arrow A1 and A2 directions) of the abutting member 68.

The gate portion 104 extends from the proximal end of the cylindrical portion 102 in the proximal end direction (arrow A2 direction). The gate portion 104 includes a pair of column portions 122 and a bridge portion 124 that bridges proximal ends of the column portions 122. The pair of column portions 122 extends along the axial direction from the cylindrical portion 102 toward the proximal end direction, and is symmetrically arranged with respect to an axial center of the cylindrical portion 102.

The bridge portion 124 is orthogonal to the axial direction of the abutting member 68 and connects a proximal end of the one column portion 122 and a proximal end of the another column portion 122. The convex portion 126 that protrudes toward the cylindrical portion 102 is provided at the center of the bridge portion 124. In the axial direction of the abutting member 68, the bridge portion 124 and the needle member 66 face each other (refer to Fig. 3).

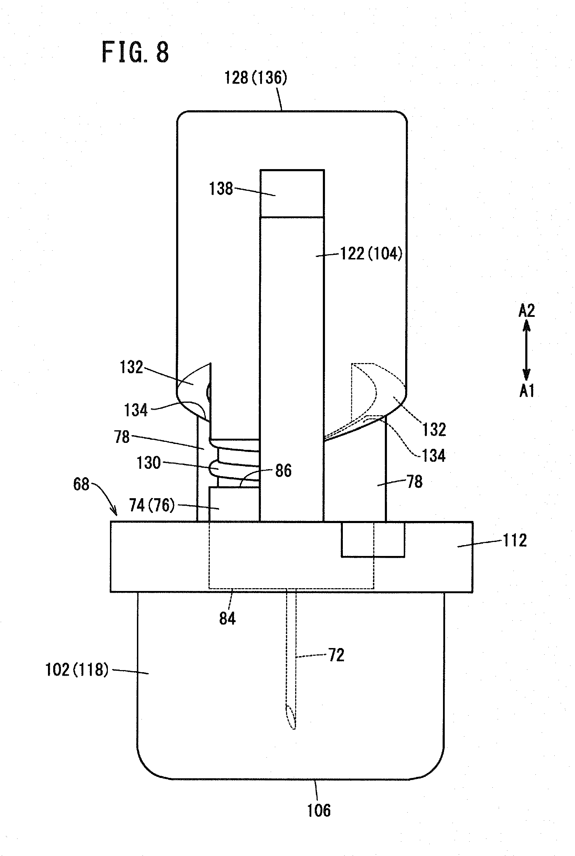

The puncture mechanism 70 causes the needle member 66 to protrude from the abutting surface 106 of the abutting member 68, in accordance with an operation of a user (for example, patient or medical personnel). As illustrated in Fig. 8, the puncture mechanism 70 includes a pusher 128 in which a pair of first inclined portions 132 is provided, a puncture biasing member 130 to be described later, and a pair of second inclined portions 134 provided on the needle hub 74 and abutting on the first inclined portion 132.

As illustrated in Fig. 3, the pusher 128 is movably disposed in the puncture direction (axial direction and arrow A1 direction) of the puncture needle 72 with respect to the housing 14. The pusher 128 is disposed in the proximal end direction (arrow A2 direction) of the abutting member 68. The pusher 128 has a bottomed cylindrical shape. The gate portion 104 of the abutting member 68 is inserted into the pusher 128. A top portion of the pusher 128 has a pressing portion 136. In the initial state of the puncture device 10, the pressing portion 136 is disposed in the second hole portion 32 of the housing cover 22.

As illustrated in Fig. 7, an outer peripheral surface of the pusher 128 has a pair of slit grooves 138 symmetrically arranged with respect to an axial center of the pusher 128. The slit groove 138 extends along the axial direction from a distal end of the pusher 128 toward a proximal end. The slit groove 138 passes through the pusher 128 in a radial direction. Each column portion 122 of the abutting member 68 is inserted into the slit groove 138. In the initial state of the puncture device 10, the proximal end of the slit groove 138 and the proximal end of the column portion 122 of the abutting member 68 are separated in the axial direction. By inserting the column portions 122 of the gate portion 104 into the pair of slit grooves 138, the pusher 128 and the abutting member 68 can relatively move along the axial direction (arrow A1 and A2 directions). That is, the pusher 128 and the abutting member 68 are prevented from relatively rotating around the axis lines of the pusher 128 and the abutting member 68.

The puncture biasing member 130 is a spring member including a coil spring. The puncture biasing member 130 is disposed between the bridge portion 124 of the abutting member 68 (refer to Fig. 3) and the hub-side receiving portion 86 of the needle hub 74. A biasing force of the puncture biasing member 130 is biased against the needle hub 74. The puncture biasing member 130 biases the needle member 66 toward the skin S of the patient. By biasing the needle member 66 toward the skin S of the patient by the puncture biasing member 130, the needle member 66 can relatively move in the puncture direction (arrow A1 direction) with respect to the abutting member 68.

As illustrated in Fig. 8, the pair of first inclined portions 132 is disposed at the distal end of the pusher 128. The first inclined portion 132 is inclined with respect to the axial direction of the pusher 128. In other words, the first inclined portion 132 is inclined with respect to the puncture direction (arrow A1 direction) of the puncture needle 72. The first inclined portion 132 is inclined so as to extend clockwise toward the distal end direction (arrow A1 direction), when the pusher 128 is viewed from the proximal end toward the distal end.

The second inclined portion 134 is disposed in each guided portion 78 of the needle hub 74. The second inclined portion 134 is disposed at the proximal end of the guided portion 78. The first inclined portion 132 of the pusher 128 can abut on the second inclined portion 134. The second inclined portion 134 is inclined with respect to the puncture direction (arrow A1 direction) of the needle member 66. That is, the second inclined portion 134 is inclined so as to extend clockwise toward the distal end direction (arrow A1 direction), when the needle member 66 is viewed from the proximal end toward the distal end direction.

The second inclined portion 134 is inclined in the same direction as the first inclined portion 132. Therefore, in the initial state of the puncture device 10 illustrated in Fig. 7, the first inclined portion 132 and the second inclined portion 134 come into surface contact with each other.

Next, an operation of the medicine solution administration device 12 including the puncture device 10 will be described.

First, in the medicine solution administration device 12, before being used (initial state), illustrated in Fig. 3, the distal end portion (protrusion portion 118) of the abutting member 68 including the abutting surface 106 protrudes toward the contact surface 26 (bonding material 25) of the housing 14.

As illustrated in Fig. 9, by bringing the contact surface 26 of the housing 14 into contact with the skin S of the patient (bond bonding material 25 to skin S), the abutting surface 106 of the abutting member 68 has contact with the skin S, and the protrusion portion 118 is pushed toward the direction (arrow A2 direction) of the housing 14 by the skin S. The abutting member 68 moves in the axial direction toward the direction of the housing 14 while compressing the following biasing member 116, and the protrusion portion 118 is housed in the space 24 of the housing 14 through the first hole portion 28.

At this time, the needle member 66 and the pusher 128 move with respect to the housing 14, together with the abutting member 68. A positional relationship between the abutting member 68 and the needle member 66 is not changed by the biasing force of the puncture biasing member 130. A positional relationship between the abutting member 68 and the pusher 128 does not change.

The movement of the needle member 66 elastically deforms the spiral portion 94 of the tube 92 to be contracted upward in the axial direction.

As the abutting member 68 moves, the pusher 128 is pushed up by the needle member 66, and the pressing portion 136 of the pusher 128 is housed in the cover member 36 through the second hole portion 32. Since the pressing portion 136 protrudes upward (arrow A2 direction) from the upper wall 22a of the housing cover 22, the user can press the pressing portion 136 toward the puncture direction (arrow A1 direction) via the covering portion 36b of the cover member 36.

Next, the user pushes the pressing portion 136 of the pusher 128 via the cover member 36 and pushes the pressing portion 136 into the housing 14. As a result, the pusher 128 moves in the puncture direction (arrow A1 direction) along the support portion 38, and the first inclined portion 132 presses the second inclined portion 134 in the puncture direction. At this time, as illustrated in Fig. 7, the locking portion 100 abuts on the engagement portion 115, and the guided portion 78 having the second inclined portion 134 is prevented from moving downward. A position of the needle member 66 in a rotation direction with respect to the abutting member 68 in the initial state illustrated in Fig. 7 is referred to as a "first position".

Therefore, when the second inclined portion 134 of the needle hub 74 is pushed in the puncture direction (arrow A1 direction) by the first inclined portion 132 of the pusher 128, the pair of guided portions 78 receives a force counterclockwise by the inclination of the first inclined portion 132 and the second inclined portion 134. As a result, the needle hub 74 does not move in the puncture direction and starts to rotate in a counterclockwise direction from the first position, around the axis line of the needle hub 74.

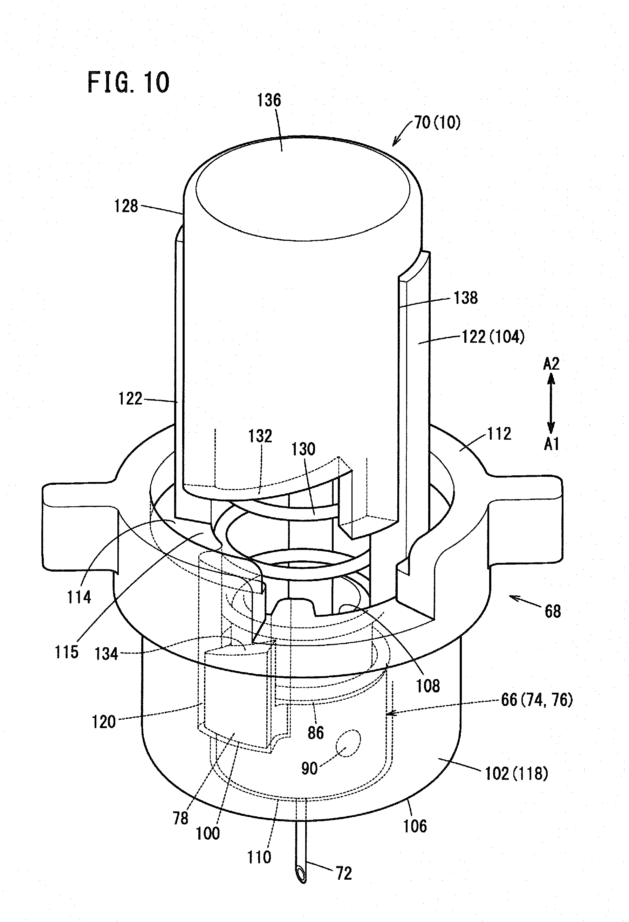

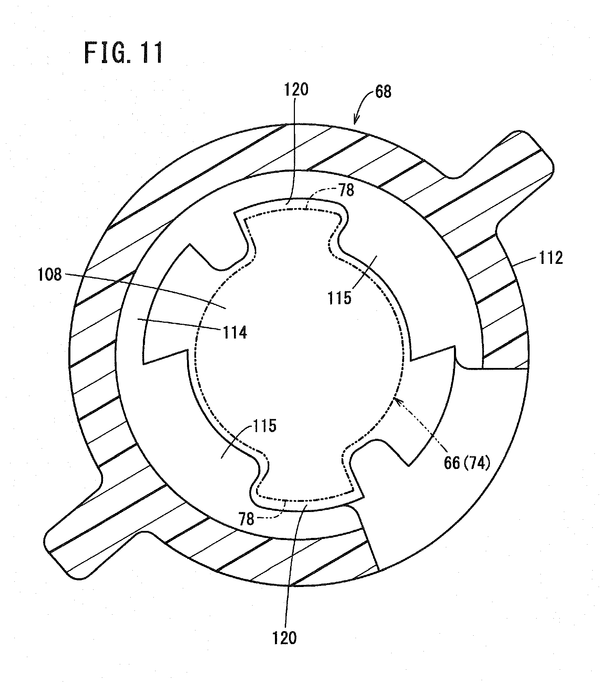

As illustrated in Figs. 10 and 11, when the needle hub 74 rotates, the pair of guided portions 78 reaches a second position facing the proximal end of the guide groove 120, and the locking portion 100 is removed from the engagement portion 115, the needle hub 74 moves in the puncture direction (arrow A1 direction) by the biasing force of the puncture biasing member 130. At this time, the guided portion 78 is inserted into the guide groove 120, the guided portion 78 is guided along the guide groove 120 in the puncture direction, and the needle hub 74 moves. A proximal end of the slit groove 138 of the pusher 128 abuts on the proximal end of the column portion 122 of the abutting member 68, and the pusher 128 is held in the puncture direction by the abutting member 68 (refer to Fig. 10).

As a result, as illustrated in Fig. 12, the needle hub 74 moves in the puncture direction (arrow A1 direction) with respect to the abutting member 68, the hub surface 84 abuts on the skin S, and the puncture needle 72 punctures the skin S of the patient. At this time, the tube 92 is elastically deformed downward along with the movement of the needle member 66 in the puncture direction.

After the puncture of the puncture needle 72 to the skin S is completed, by turning on a power supply (not illustrated) of the medicine solution administration device 12, power is supplied from the power supply unit 58 illustrated in Fig. 1 to the motor 52, and the motor 52 is driven. By driving the motor 52, each of the drive gear 60, the intermediate gear 64, and the driven gear 62 rotates, and by rotating the shaft 56, the rod 46 moves toward the distal end direction in the barrel 42. When the gasket 44 is pushed in the distal end direction by the rod 46, the medicine solution M in the medicine chamber 50 is delivered from the nozzle 48 to the tube 92. The medicine solution M is supplied to the communication path 88 of the needle hub 74 illustrated in Fig. 12, and is subcutaneously administered to the patient through the puncture needle 72.

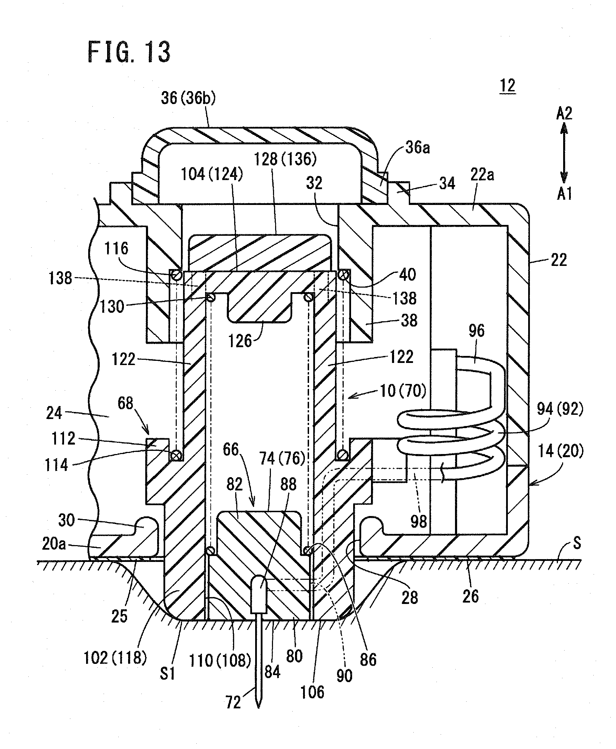

Fig. 13 illustrates a situation where, in a state where the contact surface 26 of the housing 14 has contact with the skin S of the patient and the skin S is punctured with the puncture needle 72, a facing portion S1 of the skin S facing the abutting member 68 and the needle member 66 and the contact surface 26 of the housing 14 are separated in the puncture direction (arrow A1 direction) of the needle member 66, due to a body movement of the patient or the like.

When the facing portion S1 of the skin S is separated from the contact surface 26 of the housing 14 in the puncture direction, the abutting member 68 biased by the biasing force of the following biasing member 116 relatively moves with respect to the housing 14 so as to maintain contact of the abutting surface 106 with the facing portion S1. As a result, the abutting surface 106 and the facing portion S1 are not separated in the puncture direction. By moving the abutting member 68 following the facing portion S1, the needle member 66 moves together with the abutting member 68, and the puncture of the puncture needle 72 to the skin S (facing portion S1) is maintained.

As described above, in the embodiment of the present invention, the abutting member 68 having the abutting surface 106 that has contact with the skin S of the patient is movably provided in the housing 14, and the needle member 66 including the puncture needle 72 is relatively movably provided with respect to the abutting member 68. Therefore, even in a case where a relative position between the facing portion S1, facing the abutting surface 106, of the skin S and the contact surface 26 of the housing 14 changes due to the body movement of the patient or the like in a state where the contact surface 26 of the housing 14 has contact with the skin S, the abutting member 68 relatively moves with respect to the housing 14 so as to follow the facing portion S1 of the skin S. Therefore, the puncture needle 72 of the needle member 66 is prevented from being removed from the skin S.

Since an abutting state of the abutting surface 106 of the abutting member 68 with respect to the skin S (facing portion S1) is maintained by the following biasing member 116, by including the following biasing member 116 that can bias the abutting member 68 toward the skin S of the patient in the housing 14, the removal of the needle member 66 (puncture needle 72) can be reliably prevented.

In the initial state where the abutting surface 106 of the abutting member 68 does not have contact with the skin S of the patient, since the protrusion portion 118 of the abutting member 68 protrudes from the contact surface 26 of the housing 14, the abutting surface 106 of the abutting member 68 protruding from the contact surface 26 can be reliably brought into contact with the skin S. When the contact surface 26 of the housing 14 has contact with the skin S, the abutting member 68 is pushed by the skin S so that the protrusion portion 118 is housed in the space 24 of the housing 14. Therefore, the contact surface 26 of the housing 14 can be favorably brought into contact with the skin S.

By including the puncture biasing member 130 that can bias the needle member 66 toward the skin S of the patient in the housing 14, the skin S can be reliably punctured by causing the puncture needle 72 of the needle member 66 to protrude from the abutting surface 106 of the abutting member 68 with the puncture biasing member 130.

The tube 92 used to deliver the medicine solution M to the needle member 66 and the puncture needle 72 is connected to the needle hub 74 of the needle member 66, and the tube 92 has flexibility and is housed in the space 24 of the housing 14 in a state of being bent. As a result, when the needle member 66 moves in the axial direction with respect to the housing 14, the tube 92 is elastically deformed so as to cause the tube 92 to suitably follow the needle member 66.

The above embodiment is summarized as follows.

The puncture device according to the above embodiment includes
the housing (14) having the contact surface (26) that has contact with the skin (S) of the living body,
the abutting member (68) that has the abutting surface (106) having contact with the skin, is movably provided in the housing, and of which the abutting surface can protrude from the contact surface, and
the needle member (66) that includes the puncture needle (72), can relatively move with respect to the abutting member, and of which the puncture needle can protrude from the abutting surface toward the skin, in which
when the relative position between the housing and the portion (S1), facing the abutting surface, of the skin changes in a state where the housing has contact with the skin, the abutting member relatively moves with respect to the housing so as to maintain the contact between the abutting surface and the skin.

The puncture device includes the following biasing member (116) that is provided in the housing and biases the abutting member toward the skin.

The abutting member has the protrusion portion (118) that protrudes from the contact surface of the housing when the abutting surface of the abutting member does not have contact with the skin, the protrusion portion has the abutting surface, and when the contact surface of the housing has contact with the skin, the abutting member is pushed by the skin so that the protrusion portion is housed in the housing.

The puncture device includes the puncture biasing member (130) that is provided in the housing and biases the needle member toward the skin.

The puncture device includes the puncture mechanism (70) that causes the needle member to protrude toward the skin with respect to the abutting surface of the abutting member,
the puncture mechanism includes
the pusher (128) that has the first inclined portion (132) inclined with respect to the puncture direction of the puncture needle and is movable in the puncture direction with respect to the housing,
the puncture biasing member, and
the second inclined portion (134) that is provided in the needle member, is inclined with respect to the puncture direction, and the first inclined portion abuts on the second inclined portion, in which
by moving the pusher in the puncture direction, the needle member rotates from the first position to the second position around the axis line along the puncture direction, via the first inclined portion and the second inclined portion, and
when reaching the second position, the needle member moves in the puncture direction by the biasing force of the puncture biasing member.

The needle member includes the needle hub (74) that holds the puncture needle, and
the puncture biasing member is disposed between the abutting member and the needle hub.

The tube (92) is included that is connected to the needle member and has flexibility for delivering the medicine solution (M) to the needle member,
the tube is housed in the housing in a state of being bent, and
the tube is deformed according to a displacement of the needle member with respect to the housing.

The medicine solution administration device includes
the puncture device, and
the prefilled syringe (16) that is filled with the medicine solution, is connected to the needle member of the puncture device, and delivers the medicine solution to the puncture needle.

Note that the present invention is not limited to the disclosure, and various configurations can be adopted without departing from the gist of the present invention.

## Claims

1. A puncture device comprising:
a housing having a contact surface that has contact with skin of a living body;
an abutting member that has an abutting surface that has contact with the skin, is movably provided in the housing, the abutting surface being protrudable from the contact surface; and
a needle member that includes a puncture needle and is relatively movable with respect to the abutting member, the puncture needle being protrudable from the abutting surface toward the skin, wherein
when a relative position between the housing and a portion, facing the abutting surface, of the skin changes in a state where the housing has contact with the skin, the abutting member relatively moves with respect to the housing so as to maintain the contact between the abutting surface and the skin.

2. The puncture device according to claim 1, comprising:
a following biasing member that is provided in the housing and biases the abutting member toward the skin.

3. The puncture device according to claim 1 or 2, wherein
the abutting member includes a protrusion portion that protrudes from the contact surface of the housing when the abutting surface of the abutting member does not have contact with the skin, the protrusion portion has the abutting surface, and when the contact surface of the housing has contact with the skin, the abutting member is pushed by the skin so that the protrusion portion is housed in the housing.

4. The puncture device according to any one of claims 1 to 3, comprising:
a puncture biasing member that is provided in the housing and biases the needle member toward the skin.

5. The puncture device according to claim 4, comprising:
a puncture mechanism that causes the needle member to protrude toward the skin with respect to the abutting surface of the abutting member, wherein
the puncture mechanism includes
a pusher that has a first inclined portion inclined with respect to a puncture direction of the puncture needle and is movable in the puncture direction with respect to the housing,
the puncture biasing member, and
a second inclined portion that is provided in the needle member, is inclined with respect to the puncture direction, and the first inclined portion abuts on the second inclined portion, and
by moving the pusher in the puncture direction, the needle member rotates from a first position to a second position around an axis line along the puncture direction, via the first inclined portion and the second inclined portion, and
when reaching the second position, the needle member moves in the puncture direction by a biasing force of the puncture biasing member.

6. The puncture device according to claim 4 or 5, wherein
the needle member includes a needle hub that holds the puncture needle, and
the puncture biasing member is disposed between the abutting member and the needle hub.

7. The puncture device according to any one of claims 1 to 6, comprising:
a tube that is connected to the needle member and has flexibility for delivering a medicine solution to the needle member, wherein
the tube is housed in the housing in a state of being bent, and
the tube is deformed according to a displacement of the needle member with respect to the housing.

8. A medicine solution administration device comprising:
the puncture device according to any one of claims 1 to 7; and
a prefilled syringe that is filled with a medicine solution, is connected to the needle member of the puncture device, and delivers the medicine solution to the puncture needle.
